(19) Europäisches Patentamt
European Patent Office
Office européen des brevets

(11) EP 1 077 082 A1

(12) EUROPEAN PATENT APPLICATION

(43) Date of publication:
21.02.2001 Bulletin 2001/08

(51) Int. Cl.[7]: **B01J 27/188**, C07C 51/215, C07C 5/42

(21) Application number: 00306729.5

(22) Date of filing: 07.08.2000

(84) Designated Contracting States:
**AT BE CH CY DE DK ES FI FR GB GR IE IT LI LU MC NL PT SE**
Designated Extension States:
**AL LT LV MK RO SI**

(30) Priority: **17.08.1999 US 149383 P**

(71) Applicants:
- **ROHM AND HAAS COMPANY**
  **Philadelphia, Pennsylvania 19106-2399 (US)**
- **Sunoco, Inc. (R&M)**
  **Philadelphia, PA 19103-1699 (US)**

(72) Inventors:
- **Ellis, Paul Edward**
  **Sugar Land, Texas 77479 (US)**
- **Karmakar, Swati**
  **Malvern, Pennsylvania 19355 (US)**
- **Link III, Richard David**
  **Levittown, Pennsylvania 19054 (US)**
- **Lyons, James Edward**
  **Wallingford, Pennsylvania 19086 (US)**
- **McNeal, Nneka Namono**
  **Ambler, Pennsylvania 19002 (US)**
- **Volpe, Anthony Frank, Jr.**
  **Santa Clara, California 95054 (US)**

(74) Representative:
**Buckley, Guy Julian**
**ROHM AND HAAS (UK) LTD.**
**European Operations Patent Department**
**Lennig House**
**2 Mason's Avenue**
**Croydon CR9 3NB (GB)**

# (54) Heteropolyacid/polyoxometallate catalysts

(57) Disclosed are catalysts including a heteropolyacid supported on a polyoxometallate salt having increased yields, conversions and selectivities in the conversion of alkanes. Also disclosed are methods of preparing these catalysts.

EP 1 077 082 A1

## Description

Background of the Invention

This invention relates generally to improved catalysts for a variety of chemical processes. In particular, this invention relates to improved catalysts including heteropolyacids supported on polyoxometallate salts.

In general, catalysts are well known for a variety of chemical processes, particularly for the preparation of products, such as alcohols, carboxylic acids, alkenes, alkynes, and the like, from alkanes. Typically, such catalysts are heat treated at very high temperatures for a period of time to eliminate binders or undesired impurities introduced during catalyst preparation, to form the desired compound or crystal phase, or to increase the mechanical strength of the final catalyst pellet by sintering. See, for example, C. N. Satterfield, "Heterogeneous Catalysis in Practice," McGraw-Hill, New York, 1980, p. 75.

Heteropolyacids and polyoxometallates are well known as catalysts in a variety of applications, such as the oxidation of propylene and isobutylene to acrylic and methacrylic acids, the oxidation of aromatic hydrocarbons, olefin polymerization, olefin epoxidation, hydrodesulfurizationprocesses, and the oxidation of alkanes to alcohols. For example, US 5,705,685 (Lyons et al.) discloses the use of heteropolyacids and polyoxometallates for the oxidation of alkanes to unsaturated carboxylic acids. Such heteropolyacids supported on polyoxometallate salts are particularly useful catalysts as they work at lower temperatures, that is, $<400°$ C, than other conventional catalysts. However, such heteropolyacid/polyoxometallatecatalysts suffer from relatively low yields and moderate selectivities.

In the preparation of catalysts including a heteropolyacid supported on a polyoxometallate, such catalysts are calcined at relatively low temperatures, that is, about 300° C, but are not typically heat treated at higher temperatures. Such catalysts are not typically heat treated because they work without heat treating and because they are relatively unstable and typically decompose at high temperatures, such as 400° C or greater.

Ueda et al. Chemistry Letters, (1995) 541-2, disclose the heating of molybdenum or tungsten containing heteropolyacids and one polyoxometallate, $K_3PMo_{12}O_{40}$. Such heteropolyacids were heat treated at 420° to 430° C for 2 hours under nitrogen while the polyoxometallate was heat treated at 380° C. The heteropolyacids heat treated in the presence of pyridine showed increased conversion and selectivity in the oxidation of propane to acrylic acid. The pyridine was added to maintain a reduced state of the heteropolyacid. Catalysts including heteropolyacids on polyoxometallate supports are not disclosed in this paper, nor are polyoxometallates containing cesium. Also, Ueda et al. fail to disclose a method of controlling pore size of polyoxometallate supports.

Li et al. Applied Catalysis A: General, 182 (1999), 357-363, disclose heat treatment of pyridinium salts of 12-molybdophosphonic acid, that is, a heteropolyacid. The pyridine was added to maintain a reduced state of the heteropolyacid. Li et al. do not disclose catalysts including heteropolyacids on polyoxometallate supports, nor polyoxometallates containing cesium. Also, Li et al. fail to disclose a method of controlling pore size of polyoxometallate supports.

Thus, there is a continuing need for catalysts including heteropolyacids that operate at low temperature with high yields and selectivities.

Summary of the Invention

It has been surprisingly found that heat treated heteropolyacid/polyoxometallatecatalysts increase the yields and selectivities in a variety of chemical processes, such as in the conversion of alkanes to various products, such as alkenes, unsaturated carboxylic acids, alcohols and the like. Such heat treated catalysts retain the advantages of operating at lower temperatures than other conventional catalysts. These catalysts are more attractive than other known catalysts for practical use and potential commercial interest.

In one aspect, the present invention is directed to a catalyst composition including a heteropolyacid situated on a polyoxometallate support; wherein the heteropolyacid has the formula

$$H_{(e'-bz')}G_b(X_{k'}M_{m'-x'}M^1_{x'}M^2_{n'}O_{y'})^{-e'} \qquad (I)$$

wherein G is an element selected from Groups 1-16 or an oxy ion thereof; X is an element selected from Groups 3-16; M = molybdenum, tungsten or a combination thereof; $M^1$ = vanadium; $M^2$ is a transition metal different from M and $M^1$; z' = the charge on G; b = 0 to 12; k' = 1 to 5; m' = 5 to 20;x' = 0 to 6; n'= 0 to 3; y' = 18 to 62; and e' is the charge of the polyoxometallate anion;

wherein the polyoxometallate support has the formula

$${}_aH_{(e-az)}(X_kM_{m-x}M^1_xM^2_nO_y)^{-e} \qquad (II)$$

wherein C is a cation selected from potassium, rubidium, cesium, magnesium, calcium, strontium, barium, transition metal, actinide metal, lanthanide metal, metal oxy ion, ammonium, tetraalkylammonium,pyridinium quinolinium, protonated aromatic amines, protonated aliphatic amines or mixtures thereof; X is an element selected from Groups 3-16 elements; M = molybdenum, tungsten or a combination thereof; $M^1$ = vanadium; $M^2$ is a transition metal different from M and $M^1$; z = the charge on C; k = 1 to 5; m = 5 to 20; n = 0 to 3; x = 0 to 6; y = 18 to 62; and e is the charge of the polyoxometallate anion; and
wherein the polyoxometallate support is heat treated at a temperature in the range of 385° to 600° C.

In a second aspect, the present invention is also directed to a process for preparing a catalyst composition including a heteropolyacid on a polyoxometallate support; wherein the heteropolyacid has the formula

$$H_{(e'-bz')}G_b(X_{k'}M_{m'-x'}M^1_{x'}M^2_{n'}O_{y'})^{-e'} \qquad (I)$$

wherein G is an element selected from Groups 1-16 or an oxy ion thereof; X is an element selected from Groups 3-16; M = molybdenum, tungsten or a combination thereof; $M^1$ = vanadium; $M^2$ is a transition metal different from M and $M^1$; z' = the charge on G; b = 0 to 12; k'= 1 to 5; m' = 5 to 20; x' = 0 to 6; n' = 0 to 3; y' = 18 to 62; and e' is the charge of the polyoxometallate anion;
wherein the polyoxometallate support has the formula

$$C_aH_{(e-az)}(X_kM_{m-x}M^1_xM^2_nO_y)^{-e} \qquad (II)$$

wherein C is a cation selected from potassium, rubidium, cesium, magnesium, calcium, strontium, barium, transition metal, actinide metal, lanthanide metal, metal oxy ion, ammonium, tetraalkylammonium, pyridinium, quinolinium, protonated aromatic amines, protonated aliphatic amines or mixtures thereof; X is an element selected from Groups 3-16; M is as defined above; $M^1$ = vanadium; $M^2$ is a transition metal different from M and $M^1$; z = the charge on C; k = 1 to 5; m = 5 to 20; n = 0 to 3; x = 0 to 6; y = 18 to 62; and e is the charge of the polyoxometallate anion; including the step of heating the polyoxometallate support at a temperature in the range of 385° to 600° C.

In a third aspect, the present invention is directed to a process for preparing unsaturated carboxylic acids including the steps of contacting an alkane with an oxidizing agent and a catalyst composition including a heteropolyacid on a polyoxometallate support; wherein the heteropolyacid has the formula

$$H_{(e'-bz')}G_b(X_{k'}M_{m'-x'}M^1_{x'}M^2_{n'}O_{y'})^{-e'} \qquad (I)$$

wherein G is an element selected from Groups 1-16 or an oxy ion thereof; X is an element selected from Groups 3-16; M = molybdenum, tungsten or a combination thereof; $M^1$ = vanadium; $M^2$ is a transition metal different from M and $M^1$; z' = the charge on G; b = 0 to 12; k' = 1 to 5; m' = 5 to 20; x' = 0 to 6; n' = 0 to 3; y' = 18 to 62; and e' is the charge of the polyoxometallate anion;
wherein the polyoxometallate support has the formula

$$C_aH_{(e-az)}(X_kM_{m-x}M^1_xM^2_nO_y)^{-e} \qquad (II)$$

wherein C is a cation selected from potassium, rubidium, cesium, magnesium, calcium, strontium, barium, transition metal, actinide metal, lanthanide metal, metal oxy ion, ammonium, tetraalkylammonium,pyridinium, quinolinium, protonated aromatic amines, protonated aliphatic amines or mixtures thereof; X is an element selected from Groups 3-16 elements; M = molybdenum, tungsten or a combination thereof; $M^1$ = vanadium; $M^2$ is a transition metal different from M and $M^1$; z = the charge on C; k =1 to 5; m = 5 to 20; n = 0 to 3; x = 0 to 6; y = 18 to 62; and e is the charge of the polyoxometallate anion;
wherein the heteropolyacid is present in an amount such that 100% of the surface area of the polyoxometallate is covered; and
wherein the polyoxometallate support is heat treated at a temperature in the range of 385° to 600° C.

In a fourth aspect, the present invention is directed to a process for preparing alkenes including the steps of contacting an alkane with an oxidizing agent and a catalyst composition comprising a heteropolyacid on a polyoxometallate support; wherein the heteropolyacid has the formula

$$H_{(e'-bz')}G_b(X_{k'}M_{m'-x'}M^1_{x'}M^2_{n'}O_{y'})^{-e'} \qquad (I)$$

wherein G is an element selected from Groups 1-16 or an oxy ion thereof; X is an element selected from Groups 3-16; M = molybdenum, tungsten or a combination thereof; $M^1$ = vanadium; $M^2$ is a transition metal different from M and $M^1$; z' = the charge on G; b = 0 to 12; k' = 1 to 5; m' = 5 to 20; x' = 0 to 6; n' = 0 to 3; y' = 18 to 62; and e' is the charge of the polyoxometallate anion;
wherein the polyoxometallate support has the formula

$$C_aH_{(e-az)}(X_kM_{m-x}M^1_xM^2_nO_y)^{-e} \qquad (II)$$

wherein C is a cation selected from potassium, rubidium, cesium, magnesium, calcium, strontium, barium, transition metal, actinide metal, lanthanide metal, metal oxy ion, ammonium, tetraalkylammonium, pyridinium, quinolinium, protonated aromatic amines, protonated aliphatic amines or mixtures thereof; X is an element selected from Groups 3-16 elements; M = molybdenum, tungsten or a combination thereof; $M^1$ = vanadium; $M^2$ is a transition metal different from M and $M^1$; z = the charge on C; k = 1 to 5; m = 5 to 20; n = 0 to 3; x = 0 to 6; y = 18 to 62; and e is the charge of the polyoxometallate anion;
wherein the heteropolyacid is present in an amount such that less than 100% of the surface area of the polyoxometallate is covered; and
wherein the polyoxometallate support is heat treated at a temperature in the range of 385° to 600° C.

Brief Description of the Drawings

Figure 1 shows a plot of surface area and crystallite size versus temperature under inert atmosphere.
Figures 2 shows a plot of pore volume versus average pore diameter for $Cs_3PMo_{12}O_{40}$ heat treated at various temperatures in an argon atmosphere.
Figure 3 shows a plot of pore area versus average pore diameter for $Cs_3PMo_{12}O_{40}$ heat treated at various temperatures in an argon atmosphere.

Detailed Description of the Invention

As used throughout the specification, the following terms shall have the following meanings, unless the context clearly indicates otherwise.

The term "alkane" includes linear, branched and cyclic alkanes. Likewise, the term "alkene" includes linear, branched and cyclic alkenes. All elements are referred to using the nomenclature recommended by the International Union of Pure and Applied Chemistry ("IUPAC"). As used herein, the term "transition metal" refers to an element of Groups 3-12 and includes the lanthanide and actinide series. The term "heteropolyacid/polyoxometallate catalyst" refers to a catalyst including a heteropolyacid supported on a polyoxometallate salt.

All ratios and amounts are by weight, unless otherwise noted. All numerical ranges are inclusive, unless otherwise noted. The following abbreviations are used throughout the specification: C = Centigrade; AA = acrylic acid; HPA = heteropolyacid; POM = polyoxometallate; g = gram; N = normal; mol = mole; L = liter; m = meter; Å = angstrom; cc = cubic centimeter; and ml = milliliter.

The polyoxometallates and heteropolyacids of the present invention consist of a polyhedral cage structure or framework bearing a negative charge (e.g., $[PW_{12}O_{40}]^{-3}$) which is balanced by cations that are external to the cage. When at least one of the cations is a proton, the compound is a heteropolyacid ("HPA") (e.g., $H_3[PW_{12}O_{40}]$ and $H(VO)[PMo_{12}O_{40}]$). When none of the cations are protons, but are metals such as an alkali metal, potassium, sodium, cesium or lithium, as in $K_3PW_{12}O_{40}$, or ammonium, as in $(NH_4)_3PW_{12}O_{40}$, the compound is a polyoxometallate ("POM"). When a polyoxometallate is combined with an additional acid, for example, sulfuric acid, the resulting mixture is included in the term heteropolyacid. Polyoxoanion describes the anionic cage-like portion of the compound, e.g. $[PW_{12}O_{40}]^{-3}$.

Heteropolyacids and polyoxometallates are cage-like structures with a primary, generally centrally located atom(s) surrounded by a cage framework, which framework contains a plurality of metal atoms, the same or different, bonded to oxygen atoms. The central element of heteropolyacids and polyoxometallates is different from metal atoms

of the framework and is sometimes referred to as the "hetero" element or atom; the condensed coordination elements are referred to as the "framework" elements or metals, and are ordinarily transition metals. The majority of heteropolyacids and polyoxometallates have a centrally located heteroatom ("X") usually bonded in a tetrahedral fashion through four oxygen atoms to the "framework" metals ("M"). The framework metals, in turn, (i) are usually bonded to the central atom in an octahedral fashion through oxygens ("O"), and (ii) are bonded to four other framework metals through oxygen atoms and (iii) have a sixth non-bridging oxygen atom known as the "terminal oxygen" atom. This is illustrated by Formula (III).

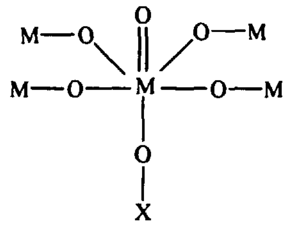

(III)

The principal framework metal, M, is any that has an appropriate cation radius and is a good oxygen pπ-electron acceptor. Typically, the framework metal is selected from molybdenum, tungsten, vanadium, niobium or tantalum. It is preferred that the framework metal is molybdenum, tungsten or vanadium.

Conventional heteropolyacids (and polyoxoanions thereof) can be described by the general formula $H_e(X_kM_nO_y)^{-e}$. In this formula, X, the central atom, is typically a Group 3-16 element, and preferably a Group 13-16 element. Suitable Group 13-16 elements include, but are not limited to: phosphorus, antimony, silicon and boron. It is preferred that the central atom, X, is phosphorus. The subscript "k" is typically from 1 to 5, and preferably 1 or 2. M is typically molybdenum, tungsten, or vanadium. The subscript "n" is typically from 5 to 20. The subscript "y" is typically from 18 to 62, and preferably about 40 to 62. The notation "e" is the negative charge on the $(X_kM_nO_y)$ polyoxoanion and will vary from case to case, but "e" is always the number of protons needed to balance the formula. In a typical Keggin heteropolyacid, k=1, n=12 and y=40 as in $H_3PMo_{12}O_{40}$ and the polyoxometallate $K_4PW_{11}VO_{40}$. In a typical Dawson heteropolyacid, k=2, n=18 and y=62 as in $H_6P_2Mo_{18}O_{62}$ and the polyoxometallate $K_6P_2W_{17}VO_{62}$.

Heteropolyacids and polyoxometallates are known to exist in a variety of structures including the Keggin, Dawson and Anderson structures. These different structures correspond to the specific geometry of particular heteropolyacid compositions and vary according to the coordination chemistry and atomic radii of the metals present. Any of these structures, or mixtures thereof, are suitable for use in the present invention.

Framework-substitutedheteropolyacids and polyoxometallates are also useful in the present invention. These compounds are heteropolyacids and polyoxometallates where certain framework atoms M (and the oxygen atoms doubly bonded to them) are replaced with transition metals. The substitution may, for example, be monosubstitution, random- or regio-disubstitution, random-or regio-trisubstitution, or higher substitutions, all of which produce effective compositions for use as the supported heteropolyacid and the polyoxometallate support in the process of the present invention. The catalysts may be further promoted by a variety of means described below. The present invention encompasses unsubstituted and substituted heteropolyacids supported on salts of unsubstituted and substituted polyoxometallates.

A typical heteropolyacid useful in making the framework-substituted compositions has the formula $H_3PMo_{12}O_{40}$. When three Mo=O units are replaced with, e.g. iron (Fe), the resulting framework substituted heteropolyacid has the formula $H_6PMo_9Fe_3O_{37}$. Thus, the general formula of the regioselectively framework-substitutedheteropolyacids described above becomes:

$$H_e(X_kM_nM^1{}_mO_y)^{-e}$$

wherein k is from 1 to 5, n is from 5 to 19, m is from 1 to 6 and y is from 18 to 62. In this formula, $M^1$ comprises one or more of zinc or any of the transition metals, namely the Group 3-10 metals of the periodic table. Preferably the transition metal is an element selected from Groups 8-10 or the first row of Groups 4-7, such as, but not limited to: iron, cobalt, nickel, ruthenium, rhodium, palladium, osmium, iridium, platinum (Groups 8-10) or titanium, vanadium, chromium, manganese (Groups 4-7, first row). Among the more preferred $M^1$ metals are iron, manganese, vanadium and combinations of nickel and iron or other transition metal. The three $M^1$ atoms do not have to be the same. However, the three $M^1$ atoms must be different from the three M atoms replaced.

By substituting oxidation-active metals, such as iron, for metals, such as molybdenum, in certain Keggin or

Dawson structures, superior catalysts, particularly for the direct oxidation of alkanes to alkenes or unsaturated carboxylic acids, such as, propylene, isobutylene, acrylic acid or methacrylic acid, may be obtained. By superior catalysts are meant catalysts that have increased conversion, yield and/or selectivity for the conversions of alkanes to alkenes. Such oxidation-active metal substitution may be framework or extra-framework, i.e. outside the framework. When such oxidation-active metals are substituted into the heteropolyacids, they may be a substitute for protons.

The heteropolyacids useful in the present invention are typically soluble in water and polar organic solvents, such as acetonitrile and alcohols, such as methanol. Such heteropolyacids are of the formula

$$H_{(e'-bz')}G_b(X_{k'}M_{m'-x'}M^1_{x'}M^2_{n'}O_{y'})^{-e'} \qquad (I)$$

wherein G is an element selected from Groups 1-16 or an oxy ion thereof; X is an element selected from Groups 3-16; M = molybdenum, tungsten or a combination thereof; $M^1$ = vanadium; $M^2$ is transition metal different from M and $M^1$; z' = the charge on G; b = 0 to 12; k' = 1 to 5; m' = 5 to 20; x' = 0 to 6; n' = 0 to 3; y' = 18 to 62; and e' is the charge of the polyoxometallate anion. Suitable elements for G include, but are not limited to: titanium, zirconium, hafnium, vanadium, niobium, tantalum, chromium, molybdenum, tungsten, manganese, rhenium, iron, cobalt, nickel, ruthenium, rhodium, palladium, osmium, iridium, platinum, copper, zinc, or combinations thereof. Suitable oxy anions of elements for G include, but are not limited to: titanyl, vanadyl, niobyl, or combinations thereof. Suitable transition elements for X include, but are not limited to: phosphorus, silicon, gallium, aluminum, arsenic, germanium, boron, cobalt, cerium, praseodymium, uranium, thorium or mixtures thereof. Suitable transition elements for $M^2$ include, but are not limited to: titanium, zirconium, hafnium, vanadium, niobium, tantalum, chromium, molybdenum, tungsten, manganese, rhenium, iron, cobalt, nickel, ruthenium, rhodium, palladium, osmium, iridium, platinum, copper, zinc or combinations thereof. It is preferred that when M is a combination of molybdenum and tungsten and the compound is a Keggin ion, x' = 0. It is also preferred that when M is molybdenum and the compound is a Keggin ion, x' = 0 to 3. It is further preferred that when M is tungsten and the compound is a Keggin ion, x' = 0 to 6.

In the above formula (e' - bz') describes the number of protons ("$H^+$") present in the heteropolyacid component of the catalyst. At a minimum, (e' - bz') is preferably greater than or equal to 0.1. In one embodiment of the invention, (e' - bz') is greater than or equal to 0.5, in another it is greater than or equal to 1. In some embodiments, bz' equals zero and the number of protons in the HPA is e'. In another embodiment, (e' - bz') is formally 0 and the protons are added by treating the system with another acid, such as sulfuric acid. Suitable heteropolyacids useful in the present invention include, but are not limited to: $H_3PMo_{12}O_{40}$, $H_3PW_{12}O_{40}$, $(VO)_{1.5}PMo_{12}O_{40}$, $(VO)_{1.5}PW_{12}O_{40}$, $(TiO)_{1.5}PMo_{12}O_{40}$, $H(VO)PMo_{12}O_{40}$, $H(VO)PW_{12}O_{40}$, $H_6PV_3Mo_9O_{40}$, $H_5PV_2Mo_{10}O_{40}$, $H_5PV_2W_{10}O_{40}$, $H_4PVMo_{11}O_{40}$, $H_4PVW_{11}O_{40}$, $RhPMo_{12}O_{40}$, $BiPMo_{12}O_{40}$, $HCrPVMo_{11}O_{40}$, $HBiPVMo_{11}O_{40}$, or combinations thereof. It is preferred that the heteropolyacid is $H_3PMo_{12}O_{40}$, $H_3PW_{12}O_{40}$, $(VO)_{1.5}PMo_{12}O_{40}$, $H(VO)PMo_{12}O_{40}$, $H_5PV_2Mo_{10}O_{40}$, $H_4PVMo_{11}O_{40}$, $RhPMo_{12}O_{40}$, $HCrPVMo_{11}O_{40}$, or $HBiPVMo_{11}O_{40}$. It is more preferred that the heteropolyacid is $H_3PMo_{12}O_{40}$, $H_3PW_{12}O_{40}$, $(VO)_{1.5}PMo_{12}O_{40}$, $H(VO)PMo_{12}O_{40}$. When the heteropolyacid is, for example, $(VO)_{1.5}PMo_{12}O_{40}$, $(TiO)_{1.5}PMo_{12}O_{40}$, $RhPMo_{12}O_{40}$, or $BiPMo_{12}O_{40}$, the necessary acid typically comes from a separate acid source, such as sulfuric acid in the $VOSO_4$ used to make $(VO)_{1.5}PMo_{12}O_{40}$. Such amount of acid is sufficient for the present invention.

The polyoxometallates useful as supports in the present invention are typically water insoluble and are those of the formula

$$C_aH_{(e-az)}(X_kM_{m-x}M^1_xM^2_nO_y)^{-e} \qquad (II)$$

wherein C is a cation selected from potassium, rubidium, cesium, magnesium, calcium, strontium, barium, transition metal, actinide metal, lanthanide metal, metal oxy ion, ammonium, tetraalkylammonium,pyridinium, quinolinium, protonated aromatic amines, protonated aliphatic amines or mixtures thereof; X is an element selected from Groups 3-16; M, the first framework metal, is molybdenum, tungsten or a combination thereof $M^1$, the second framework metal, is substituted for the first framework metal and is vanadium; $M^2$, the third framework metal, is different from M and $M^1$ and is independently transition metal; z' = the charge on C; k = 1 to 5; m = 5 to 20; n = 0 to 3; x = 0 to 6; y = 18 to 62; and e' is the charge of the polyoxometallate anion. Suitable transition metals useful for C include, but are not limited to: vanadium, chromium, lanthanum, manganese, iron, cobalt, ruthenium, copper and the like. Suitable metal oxy ions useful for C include, but are not limited to: oxy ions of vanadium, oxy ions of chromium, oxy ions of uranium, and the like. It is preferred that C is potassium, rubidium, cesium, magnesium, calcium, strontium, barium, lanthanum, ammonium, tetraalkylammonium, pyridinium, quinolinium, protonated aromatic amines, protonated aliphatic amines, or mixtures thereof, and more preferably cesium. Suitable transition elements for X are those useful as X in the heteropolyacids described above. Suitable transition elements useful for $M^2$ are those described above for the heteropolyacids. When M is molybdenum and the compound is a Keggin ion, it is preferred that x = 0 to 3. When M is tungsten and the compound is a Keggin ion, it is preferred that x = 0 to 6. When "az" equals "e", there are no protons present in the polyoxometallate support, which is preferred.

Suitable polyoxometallate supports useful in the present invention include wide pore salts, for example wide pore cesium salts of the various substituted polyoxometallates described in US Ser. No. 08/565,206, herein incorporated by reference to the extent it teaches the preparation of such salts. It is preferred that the polyoxometallate support is $Cs_{3+x}(PM_{12-x}V_xO_{40})$, where x = 0 to 3 and M = molybdenum or tungsten. Suitable preferred polyoxometallate supports include $Cs_3(PMo_{12}O_{40})$, $Cs_4(PMo_{11}VO_{40})$, $Cs_5(PMo_{10}V_2O_{40})$, $Cs_6(PMo_9V_3O_{40})$, $Cs_3(PW_{12}O_{40})$, $Cs_4(PW_{11}VO_{40})$, $Cs_5(PW_{10}V_2O_{40})$, $Cs_6(PW_9V_3O_{40})$, or combinations thereof. It is more preferred that the polyoxometallate support is $Cs_3(PMo_{12}O_{40})$, $Cs_3(PW_{12}O_{40})$ or combinations thereof.

The polyoxometallate supports of the catalysts of the present invention may have large (i.e. wide) or small pores or a mixture of pore sizes. It is preferred that the polyoxometallate support has large pores. For example, the polyoxometallate support preferably has pore volumes in the range from 0.01 to 0.25 ml/g and a pore size distribution in which more than approximately 60% of the pore volume is due to pores having a median pore radius of greater than or equal to approximately 75 Å, preferably greater than or equal to approximately 100 Å, more preferably greater than or equal to approximately 150 Å, still more preferably greater than or equal to approximately 200 Å. More preferably, the support has pore volumes in the range from 0.05 to 0.25 ml/g and a pore size distribution in which more than approximately 60% of the pore volume is due to pores having a median pore radius of greater than or equal to approximately 75 Å. In a preferred embodiment, the support material has pore volumes in the range from 0.01 to 0.25 ml/g and a pore size distribution in which more than approximately 80% of the pore volume is due to pores having a median pore radius of greater than or equal to approximately 200 Å; more preferably, the support material has pore volumes greater than 0.15 ml/g and a pore size distribution in which more than approximately 80% of the pore volume is due to pores having a median pore radius of greater than or equal to approximately 200 Å.

In a more preferred embodiment, the pores in the support have pore radii of greater than 75 Å and pore volumes greater than 0.05 ml/g; more preferably, the pore radii are greater than 100 Å, and independently, the pore volumes are greater than 0.1 ml/g. It has been found that supports with pore volumes greater than 0.02 ml/g result in catalysts with superior catalytic performance, provided the pores are wide (i.e., radii greater than approximately 75 Å). These polyoxometallates may be further modified by pretreatment with water, such as steam, washing with various solvents, and by formation in the presence of vanadyl acetylacetonate or $VOSO_4$. Such supports may also be ground to modify pore sizes.

The polyoxometallate or heteropolyacid component used in the present invention may contain second framework metals which have been substituted into the framework thereof, replacing an equivalent number of the first framework metals. Such substituting metals include, but are not limited to: titanium, zirconium, hafnium, vanadium, niobium, tantalum, chromium, molybdenum, tungsten, manganese, rhenium iron, cobalt, nickel, ruthenium, rhodium, palladium, osmium, iridium, platinum, copper, zinc or combinations thereof. The second framework metal, $M^1$, is different from the first framework metal, M. When there are more than one $M^1$ atoms, each $M^1$ is bound through an oxygen atom to another $M^1$.

The atoms which are replaced in such substitution include, but are not limited to: molybdenum, tungsten, vanadium or combinations thereof. The number of framework atoms replaced may be from 1 to 3 or more, and the substituting metals, which are different from the replaced metal, may each be the same metal, for example iron, or may be different from each other, for example two or three different metal atoms; e.g., one iron atom may replace one tungsten atom; two iron atoms may replace two tungsten atoms; three iron atoms may replace three tungsten atoms; two atoms, different from each other, for example iron and cobalt, may replace two tungsten atoms; three atoms, different from each other, for example iron, cobalt and nickel, may replace three tungsten atoms; two atoms of iron and one atom of cobalt may replace three tungsten atoms; and so on. Replacement of three framework atoms of a POM or HPA by three atoms, different from the framework atom, two of which replacing atoms are selected from the group consisting of iron, chromium, manganese or ruthenium, and the third of which is different from the two just referred to and is a transition metal, is disclosed in U.S. 5,091,354 (Lyons et al.).

Examples of such heteropolyacids include $H_6PW_9Fe_3O_{37} \cdot NaN_3$ wherein phosphorus (P) is the heteroatom, tungsten (W) is the first framework metal, and iron (Fe) is the second framework metal; $H_7PW_9Fe_2MO_{37} \cdot NaN_3$, wherein phosphorus (P) is the heteroatom, tungsten (W) is the first framework metal, and iron (Fe) and "M" are the second framework metals, M being variously nickel, manganese, cobalt, zinc; and $H_7PW_9Cr_3O_{37} \cdot NaN_3$, wherein phosphorus (P) is the heteroatom, tungsten (W) is the first framework metal, and chromium (Cr) is the second framework metal. Examples of such heteropolyacids, include $H_3PW_{10}M_2O_{40}$, where M is titanium, zirconium, niobium, tantalum, manganese, iron, cobalt, nickel or copper. These compositions are useful as the POM and/or HPA component of the supported catalysts of the present invention.

The polyoxometallate or the heteropolyacid, or both, may independently comprise (1) at least 6 atoms of a first framework metal or metals comprising molybdenum, tungsten, vanadium or combinations thereof and (2) at least one atom of a second framework metal or metals comprising a transition metal other than molybdenum, tungsten or

vanadium. When there is more than one second framework metal, they may comprise a combination of the available transition metals.

In one embodiment, the polyoxometallate and/or the heteropolyacid used in the present invention comprises 9 to 11 atoms of a first framework metal selected from the group consisting of molybdenum, tungsten and vanadium, and 1 to 3 atoms of a second framework metal such as titanium, zirconium, vanadium, niobium, tantalum, chromium, molybdenum, tungsten, manganese, rhenium, iron, cobalt, nickel, ruthenium, rhodium, palladium, osmium, iridium, platinum, copper or zinc, which second metal is a transition metal different from the first framework metal. The second framework metals, $M^1$, are site-specific, regioselective substitutions wherein each $M^1$ is bound through an oxygen atom to another $M^1$.

The heteropolyacids useful in the present invention are commercially available or may be prepared by a variety of methods known in the art. General syntheses of the polyoxometallates and heteropolyacids useful in the present invention are described in Pope et. al., *Heteropoly and Isopoly Oxometallates*, Springer-Verlag, New York (1983). Typically, heteropolyacids are prepared by dissolving the desired metal oxides in water, adjusting the pH to approximately 1 to 2 with acid, such as hydrochloric acid, to provide the necessary protons, and then evaporating water until the desired heteropolyacid precipitates. As an example, the heteropolyacid $H_3PMo_{12}O_{40}$ can be prepared by combining $Na_2HPO_4$ and $Na_2MoO_4$, adjusting the pH with sulfuric acid, extracting with ether, and crystallizing the resulting heteropolyacid in water. Also, vanadium-substituted heteropolyacids may be prepared according to the method described in V. F. Odyakov, et. al., *Kinetics and Catalysis*, 1995, vol. 36, p. 733. The $Cs_3PMo_{12}O_{40}$ support can be prepared, as further described below, by treating the above heteropolyacid with cesium carbonate and collecting the resulting precipitated product.

The heteropolyacids useful in the present invention may be promoted by various means including preparing the heteropolyacid in the presence of vanadyl acetylacetonate or the like. In addition, exchange of iron or other transition metals, actinide and lanthanide metals, and other groups, G, has been found to promote the activity of the heteropolyacids of the catalysts used in the process of the present invention.

Typically, the polyoxometallate support component of the catalyst may be prepared by adding a soluble salt of the desired cation, for example $Cs_2CO_3$ for a cesium salt support, to the desired soluble heteropolyacid, for example $H_3(PMo_{12}O_{40})$, to form the insoluble polyoxometallate, for example $Cs_3(PMo_{12}O_{40})$. The polyoxometallates may be isolated by any means, such as by filtration or solvent evaporation, and preferably by solvent evaporation. Certain polyoxometallate precipitates may be isolated without need for an evaporation step. However, in many cases the precipitate is very fine and isolation is preferably done by evaporation of water. The desired proportion of the metal oxides may vary somewhat from the theoretical amount required for the desired product.

The salt solution is preferably added slowly into the heteropolyacid solution to precipitate the cation heteropolymetallate salt. The following reaction exemplifies the process:

$$3\ Cs_2CO_3 + 2\ H_3(PMo_{12}O_{40}) \rightarrow 2\ Cs_3(PMo_{12}O_{40}) + 3\ H_2O + 2\ CO_2$$

The precipitation may be performed at an elevated temperature (e.g., 60 - 65° C) and $CO_2$ is evolved during the reaction. The resulting polyoxometallate salt forms a fine suspension in water and may be evaporated to dryness, for example by rotary evaporation, or by heating, such as at 50 to 70 °C or below. The dried material may be calcined/heat treated (e.g., at 300° C).

The method of preparation of the polyoxometallates can influence the surface area, the pore volume and the pore size distribution ("PSD") of the polyoxometallate salts. For example, slow addition of the cation salt to the heteropolyacid solution results in a material with few small pores and many large pores. In contrast, rapid addition of the cation salt yields a broad PSD with many small pores and some intermediate and large pores. For the present invention, slow addition to form mainly large pores is preferable; for example, at a rate of 2 ml/minute, particularly when using solution concentrations of approximately 0.1 mol/L. More preferably, particularly for the preparation of large quantities of material, the solutions of the cation salt and the heteropolyacid may be added simultaneously to a reaction vessel. The salt solution may have a concentration in the range of from about 0.05 to 1 mol/L, preferably 0.1 to 0.2 mol/L. The heteropolyacid solution may have a concentration in the range of from about 0.05 to 1 mol/L, preferably 0.1 to 0.2 mol/L, and more preferably 0.1 mol/L.

A further factor influencing the PSD is the temperature of the reaction medium during the precipitation step. Precipitation at room temperature yielded a narrow PSD with a median pore radius of about 90 Å, whereas precipitation at 65° C was found to result in a broader PSD with a greater median pore radius (≥ 120 Å).

Additionally, it has been found that aging of the slurry containing the polyoxometallate salt, followed by slow evaporation to dryness, is beneficial to the production of large pore materials. Preferably, the slurry is allowed to remain at room temperature or at a temperature in the range from approximately 35° C to 45° C for an extended period of time and is then slowly dried. The aging and drying process may extend for a period of 1 to 3 days or longer. Also, the use of excess cation salt (relative to the stoichiometric amount) promotes formation of the desired large-pore support material. While the support material can be prepared using stoichiometric ratios of starting materials, it is preferred to use a slight excess of the cation salt.

The preparation of heteropolyacids and polyoxometallates with random framework-metal substitution, such as $H_7(PMo_8V_4O_{40})$; $K_6(SiMo_{11}MnO_{39})$ and $K_5(PW_{11}VO_{40})$, is known. For example, $K_5(PW_{11}VO_{40})$ may be prepared by dissolving 45.0 g of 12-tungstophosphoricacid, $H_3(PMo_{12}O_{40})$, in 105 ml of water. With stirring, the pH is adjusted to about 5.2 with potassium bicarbonate. The mixture is then heated to 70 °C and 6.0 g of vanadyl sulfate ($VOSO_4$) in 15 ml water is added. The solution is cooled and potassium chloride is added to precipitate the desired $K_5(PW_{11}VO_{40})$.

The preparation of regiospecific, trilacunary framework-substituted heteropolyacids or polyoxometallates may also be useful in the present invention. Suitable regiospecific, trilacunary framework-substitutedheteropolyacids or polyoxometallates are described in US Pat. No. 4,898,989, herein incorporated by reference to the extent it teaches the preparation of such substituted compounds.

The HPAs and POMs useful in the present invention may be prepared separately and then combined or, alternatively, may be prepared together. It is preferred that the HPAs and POMs are prepared separately and then combined. In an alternative embodiment, the catalysts useful in the present invention may be prepared by generating the heteropolyacid in situ on the surface of the polyoxometallate support by treating the polyoxometallate with a strong, proton containing acid. Any strong, proton containing acid is suitable, and preferably an inorganic acid. Suitable acids include, but are not limited to: hydrochloric acid, hydrobromic acid, sulfuric acid and nitric acid.

The catalysts of the present invention include a heteropolyacid supported on a polyoxometallate salt ("HPA/POM catalysts"). Such catalysts useful in the present invention are those having the formula

$$H_{(e'-bz')}G_b(X_{k'}M_{m'-x'}M^1_{n'}M^2_{n'}O_{y'})^{-e'} / C_aH_{(e-az)}(X_kM_{m-x}M^1_xM^2_nO_y)^{-e} \qquad (IV)$$

wherein the individual components are as described in formulae (I) and (II) above. It is preferred that the HPA/POM catalyst is

$(VO)_{1.5}PMo_{12}O_{40}/Cs_3(PMo_{12}O_{40})$, $(VO)_{1.5}PMo_{12}O_{40}/Cs_3(PW_{12}O_{40})$,
$H(VO)PMo_{12}O_{40}/Cs_3(PMo_{12}O_{40})$,
$H_5PV_2Mo_{10}O_{40}/Cs_3(PMo_{12}O_{40})$,$H_5PV_2Mo_{10}O_{40}/Cs_3(PW_{12}O_{40})$,
$H_4PVMo_{11}O_{40}/Cs_3(PMo_{12}O_{40})$, $H_4PVMo_{11}O_{40}/Cs_3(PW_{12}O_{40})$,
$RhPMo_{12}O_{40}/Cs_3(PMo_{12}O_{40})$, $HCrPVMo_{11}O_{40}/Cs_3(PMo_{12}O_{40})$,
$HBiPVMo_{11}O_{40}/Cs_3(PMo_{12}O_{40})$, $H_4(PMo_{11}VO_{40})/Cs_3(PMo_{12}O_{40})$,
$H_3(PMo_{12}O_{40})/Cs_3(PMo_{12}O_{40})$, $H_3(PMo_{12}O_{40})/Cs_3(PW_{12}O_{40})$,
$H_3(PW_{12}O_{40})/Cs_3(PMo_{12}O_{40})$, or $H_3(PW_{12}O_{40})/Cs_3(PW_{12}O_{40})$, and more preferably
$H_3(PMo_{12}O_{40})/Cs_3(PMo_{12}O_{40})$, $H_3(PMo_{12}O_{40})/Cs_3(PW_{12}O_{40})$,
$H_3(PW_{12}O_{40})/Cs_3(PMo_{12}O_{40})$ or $H_3(PW_{12}O_{40})/Cs_3(PW_{12}O_{40})$.

The amount of the heteropolyacid supported on the polyoxometallate salt depends upon the desired catalytic activity. For example, catalysts of the present invention having 100% of the surface area of the polyoxometallate covered by heteropolyacid are useful in the conversion of alkanes to unsaturated carboxylic acids. Catalysts of the present invention having less than 100% of the surface area of the polyoxometallate covered by heteropolyacid are useful in the conversion of alkanes to alkenes. Thus, the any amount of heteropolyacid is suitable that catalyzes the desired reaction.

The HPA/POM catalysts of the present invention show improved catalytic activity, such as increased yields and selectivities, after heat treatment of the POM support. The catalysts of the present invention may be prepared by first heat treating the POM support followed by supporting the HPA on the POM. It is preferred that the catalysts of the present invention are heat treated after preparation, that is, after the HPA has been supported on the POM. Such heat treatment typically includes heating the catalyst for a period of time at a temperature in the range of 385° to 600° C, preferably in the range of 390° to 470°, and more preferably in the range of 410° to 470° C. Such heat treatment is typically up to 24 hours in duration, but may be longer. It is preferred that the heat treatment is up to 8 hours in duration, more preferably 4 hours, and most preferably 2 hours. It will be appreciated by those skilled in the art that longer heat treatment times may be used as long as the catalyst does not decompose. Thus, for less stable catalysts, longer heat treatment times may be employed if lower heat treatment temperatures are used. Typically, the heat treatment of the present invention is performed by a variable ramping of the temperature, such as 2° C per minute, until the desired temperature is achieved. In an alternate embodiment, the HPA/POM catalysts are heat treated more than once. For example, a HPA/POM catalyst of the present invention may be heat treated for repetitive short periods, such as 2 or more heat treatments at 410° to 450° C for 15 to 60 minutes each. The heat treatments of the present invention may be performed under inert or oxygen containing atmospheres. Suitable heat treatment atmospheres include, but are not limited to: air, oxygen, nitrogen, argon and combinations thereof. It will be appreciated that more than one atmosphere may be used, such as a first heat treatment under nitrogen, followed by air or oxygen.

The heat treatments of the present invention may be carried out in any manner. The polyoxometallate supports of the present invention may be heat treated prior to placing them in a catalytic reactor, heat treated in a catalytic reactor prior to use or heat treated in a catalytic reactor during use. In one embodiment, the HPA/POM catalysts of the present invention are placed in a reactor and heat treated without the presence of a reactive gas, such as alkanes. The reactor and catalyst are then cooled. Once cooled, the reactor and catalyst are then heated to a suitable temperature for the desired conversion of the reactive gas and the gas is then fed into the reactor. When the polyoxometallates of the present invention are heat treated during use, the catalysts are placed in a catalytic reactor and a reactive gas is fed into the reactor as the temperature of the reactor is raised to the desired heat treatment temperature. After such heat treatment, the reactor temperature is lowered to a suitable temperature for the desired conversion of the reactive gas and the reaction is continued.

The HPA/POM catalysts of the present invention having a heat treated polyoxometallate support are useful in catalyzing all reactions where non-heat treated HPA/POM catalysts are useful. For example, the HPA/POM catalysts of the present invention having a heat treated polyoxometallate support are useful in the oxidation of alkanes to alcohols, oxidation of alkanes to unsaturated carboxylic acids, oxidative dehydrogenation of alkanes to alkenes, oxidation of aromatic hydrocarbons, olefin polymerization, olefin epoxidation, hydrosulfurization processes, and oxidation of alkenes. The catalysts of the present invention are preferably used in the oxidation of alkanes to alcohols, oxidation of alkanes to unsaturated carboxylic acids, oxidative dehydrogenation of alkanes to alkenes and oxidation of alkenes.

The catalysts of the present invention are particularly useful in the conversion of alkanes to unsaturated carboxylic acids or alkenes. When a catalyst includes a heteropolyacid as a complete surface layer on a polyoxometallate support, i.e. the heteropolyacid covers 100% of the surface area of the support, alkanes are converted to unsaturated carboxylic acids. For example, propane is converted to acrylic acid and isobutane is converted to methacrylic acid. When the polyoxometallate support of such catalyst is heat treated according to the present invention, the yield of unsaturated carboxylic acid, as well as the selectivity of the conversion, increases. When a catalyst includes a heteropolyacid in an amount less than a complete surface layer on a polyoxometallate support, i.e. the heteropolyacid covers less than 100% of the surface area of the support, alkanes are converted to alkenes. When the polyoxometallate support of such catalyst is heat treated according to the present invention, the yield of alkene, as well as the selectivity of the conversion, increases.

When the catalysts of the present invention are used in the conversion of alkanes to alkenes, any amount of heteropolyacid may be used such that less than 100% of the surface area of the polyoxometallate support is covered. It is preferred that the amount of the heteropolyacid covers 0.5 to 75% of the surface area of the polyoxometallate support, more preferably 1 to 70% of the surface area, and most preferably 3 to 50% of the surface area.

For example, when the polyoxometallate support is $Cs_3PMo_{12}O_{40}$ having a surface area of 80 $m^2/g$, the amount of the heteropolyacid useful in the present invention is less than 20 mol%, and preferably less than 10 mol% (approximately 50% of the surface area covered). When the polyoxometallate support is $Cs_3PW_{12}O_{40}$ having a surface area of 110 $m^2/g$, the amount of the heteropolyacid useful in the present invention is less than 25 mol%, and preferably less than 12 mol% (approximately 50% of the surface area covered).

The catalysts useful in the present invention include a heteropolyacid on a polyoxometallate support which is heat treated, as described above. The supported catalyst including a heteropolyacid supported on a polyoxometallate salt may be prepared, for example, by incipient wetness techniques in which a solution of heteropolyacid is sprayed on a solid support matrix and then dried, or by adding support material to a solution of heteropolyacid and evaporating the solution to dryness, or by dry grinding the heteropolyacid with the polyoxometallate support. The heteropolyacid may be dissolved in water or other solvent, such as acetonitrile.

The following process illustrates the catalyst preparation using incipient wetness technique. The desired amount of heteropolyacid is dissolved in solvent (typically water or acetonitrile). After the heteropolyacid, together with precursors of groups G, if desired, are dissolved, the solution is sprayed evenly on the support material and the supported catalyst is dried, for example at 80° C for 8 hours when using water, or 50° C for 8 hours when using acetonitrile. Repeated spraying and drying steps may used to modify dispersion characteristics. The final supported catalyst material may be heat treated. If heat treated, the heat treatment temperature is preferably between 250° C and 450° C, and is not so severe as to damage the catalyst. The heat treatment may be performed, for example, at 275° C for 3 to 6 hours, or at 420° C for 1 to 2 hours.

When the catalyst of the present invention is to be used for the conversion of alkanes to alkenes and is prepared in situ by treating a polyoxometallate with a strong, proton containing acid, any amount of acid may be used as long as the resulting heteropolyacid covers less than 100% of the surface area of the polyoxometallate support.

In one embodiment of the invention, the supported heteropolyacid may be pre-treated with water, such as steam. The catalyst is prepared by exposure to air saturated with water vapor or steam for approximately 48 hours. The

hydrated catalyst may comprise about 5 to 30 weight percent water. This pretreatment of the catalyst by hydration may enhance catalytic activity.

Other pretreatment methods, such as pretreatment with amines, may be used successfully with the catalysts of the present invention. Suitable amines include pyridine, 2,2'-bipyridine, quinoline, and pyridine N-oxide. It is preferred that the catalysts of the present invention are free of pyridine.

In the alkane conversion process of the present invention, an alkane is contacted with an oxidizing agent and a heat treated catalyst of the present invention. As described above, when the heteropolyacid is present in an amount such that 100% of the surface area of the polyoxometallate support is covered, the catalyst is suitable for the conversion of alkanes to unsaturated carboxylic acids. Likewise, when the heteropolyacid is present in an amount such that less than 100% of the surface area of the polyoxometallate support is covered, the catalyst is suitable for the conversion of alkanes to alkenes.

The alkanes useful in the present invention may be linear, branched, or cyclic and are any which are gaseous at the temperature of the reaction. It is preferred that the alkanes useful in the present invention are gaseous at 370° C, and more preferably gaseous at 225° C. Thus, alkanes having 2 to 20 carbon atoms may be used successfully in the present process. The alkanes may be single compounds or mixtures of compounds. The purity of the alkanes is not critical, although it is preferable to avoid the presence of compounds which may poison the catalyst. Suitable alkanes include, but are not limited to: ethane, propane, butane, pentane, cyclopentane, hexane, cyclohexane, heptane, octane, cyclooctane, decane, dodecane, tetradecane, hexadecane and mixtures thereof. As a result, the feedstock for the present process may, in addition to the alkane or alkanes of interest, further comprise methane or ethane as well as impurities such as air or carbon dioxide. In the conversion of alkanes to alkenes, the preferred alkanes have 2 to 12 carbon atoms, and more preferably 2 to 8 carbon atoms. Suitable preferred alkanes useful in the conversion to alkenes include, but are not limited to: ethane, propane, n-butane, iso-butane, pentane, iso-pentane, hexane, iso-hexane, ethylhexane, cyclopentane, cyclohexane and mixtures thereof. Especially preferred alkanes include ethane, propane and isobutane. In the conversion of alkanes to unsaturated carboxylic acids, the preferred alkanes have 3 to 12 carbon atoms, and more preferably 3 to 8 carbon atoms. Suitable preferred alkanes useful in the conversion to unsaturated carboxylic acids include, but are not limited to: propane, n-butane, iso-butane, pentane, iso-pentane, hexane, iso-hexane, ethylhexane, cyclopentane, cyclohexane and mixtures thereof. More preferred alkanes useful in the conversion to unsaturated carboxylic acids include propane and isobutane.

Suitable oxidizing agents, or oxidants, useful in the alkane conversion process of the present invention include, but are not limited to: air, molecular oxygen, hydrogen peroxide, nitrogen oxides and mixtures thereof. It is preferred that the oxidizing agent is air, molecular oxygen or nitrogen oxides, and more preferably the oxidizing agent is air. The amount of the oxidizing agent useful in the present invention is any amount sufficient to oxidize the alkane. For example, the amount of oxidizing agent may be any amount up to or greater than the stoichiometric amount, based on the amount of alkane. Furthermore, by controlling the amounts of oxidizing agent and alkane, the catalyst may be kept oxidized or reduced and the lifetime and reactivity of the catalyst may be controlled.

It will be appreciated by those skilled in the art that the alkane conversion process of the present invention may be carried out under a variety of conditions. The conditions necessary are generally those under which the oxidizing agent functions to oxidize the alkane to an unsaturated carboxylic acid or alkene. Thus, it is preferred that the process of the present invention is run under oxidizing conditions.

The alkane conversion process of the invention is preferably carried out in the vapor phase. In the process of the present invention, an alkane, an oxidant, optionally an inert diluting gas and optionally gaseous promoters and/or modifiers are fed into a reactor. Suitable inert gases include, but are not limited to: nitrogen, argon, helium or the like. Preferably, the feedstock is an alkane gas. The alkane, oxidant and optional diluting gas may be mixed prior to being fed into the reactor or may be mixed in the reactor.

The alkane conversion process of the present invention may be carried out in the presence or absence of steam, and preferably in the presence of steam. When an inert, diluting gas is used in the process of the invention, determination of the molar ratio of alkane, oxidant, diluting gas and water (steam), if present, in the starting reaction gas mixture is within the ability of the skilled practitioner in the art. Determination of the gas space velocity used in the process of the invention is within the ability of the skilled practitioner in the art.

The temperature used in the process of the invention is that which favors the formation of unsaturated carboxylic acids or alkenes as reaction products. The conversion of alkanes to unsaturated carboxylic acids or alkenes according to the process of the present invention is carried out at a temperature in the range from 225° C to 450° C. The process of the invention is typically performed at a temperature of at least about 225° C, and preferably at least about 275° C, and below that which will cause an undesirable level of decomposition of the alkane to carbon oxide and water and/or decomposition of the catalyst. Generally, the temperature is not above 450° C, more preferably not above 400° C. Thus, a preferred temperature range is from 275° C to 400° C. It will be appreciated that when higher process temperatures are used, such as >400° C, tungsten containing catalysts are more stable and are therefore preferred for such high temperature processes. The determination of the most desirable temperature for a given reaction and given

catalyst within the scope of the invention is within the ability of the person skilled in the art.

The pressure used in the process of the invention is not critical, but is important. For example, the pressure used in the present process may affect the selectivity of alkene formation. The process may be successfully carried out at atmospheric pressure. Other pressures may be used, and the determination of the most desirable pressure for a given reaction within the scope of the invention is within the ability of the person skilled in the art.

The process of the invention may be carried out in any suitable reactor configuration. For example, the reaction may be performed in a fixed-bed, moving bed, ebullating bed reactor, or other as is within the ability of the person skilled in the art to determine.

It will be appreciated that unreacted alkane from the process of the present invention may be recycled and passed through the reactor one or more times. Such a recycle has the advantage of increasing the yield of alkene produced. It will be further appreciated that the process of the present invention may be combined with a process using unsaturated carboxylic acids or alkenes as feedstock, such as processes for the polymerization of unsaturated carboxylic acids or production of unsaturated carboxylic acids, alcohols or the like from alkenes. In such cases, the process of the present invention may be used to produce alkenes, which can then be reacted directly to form such products as unsaturated carboxylic acids, such as acrylic acid or methacrylic acid, or alcohols. In an alternative embodiment, the catalysts of the present invention may be combined with one or more additional catalysts. Such combined catalyst systems have the advantage that conversion of alkanes to unsaturated carboxylic acids or alkenes and then conversion of these products to other reaction products may be performed within one reactor or reactor system.

As the alkanes useful in the alkane conversion process of the present invention increase in carbon chain length, it is preferred that the pore size of the polyoxometallates also increase. For example, when alkanes having more than about 8 carbon atoms are used in the process of the present invention, it is preferred that the polyoxometallate supports have large pores.

Catalysts having polyoxometallates containing a majority of large pores are prepared by first preparing polyoxometallates containing large pores and then combining heteropolyacids with such polyoxometallates. The preparation of such large pore polyoxometallates is described above. However, such preparatory methods do not provide polyoxometallates having exclusively large pores. Rather, such methods provide polyoxometallates having a median pore size that is larger, meaning small pores are still present. It has been surprisingly found that pore sizes of HPA/POM catalysts can be controlled by heat treating, that is, HPA/POM catalysts having small pores are converted into HPA/POM catalysts having an even greater proportion of large pores.

It has also been found that pore sizes of polyoxometallate salts alone can be controlled by heat treating, that is, polyoxometallate salts having small pores are converted into polyoxometallate salts having a greater proportion of large pores. Such a method of preparing large pore polyoxometallate salts may have advantages over known methods of preparation. For example, it allows the preparation of catalysts having different compositions in the support and the coating, such as a tungsten-based polyoxometallate support coated with a molybdenum-based heteropolyacid. Such a method also allows the preparation of catalysts in which the coated species is sensitive to high temperatures.

Figure 1 illustrates the change in surface area (in $m^2/g$) and crystallite size, in angstroms, with heat treatment temperature for a cesium polyoxometallate, $Cs_3PMo_{12}O_{40}$, under inert atmosphere. Crystallite size was determined from the XRD line width (20° 2-theta) compared to internal standards. There was an excellent correlation between increasing crystallite size and decreasing surface area, reflecting major changes in the tertiary structure with increasing temperature.

Figures 2 and 3 show plots of pore volume and pore area versus pore diameter, respectively, for $Cs_3PMo_{12}O_{40}$ heat treated at various temperatures in an argon atmosphere. The pore diameter is reported in Å, the pore volume is reported in cc/g and the pore area is reported in $m^2/g$. These figures clearly show that as the heat treatment temperature is increased, the surface area decreases and the pore size distribution shifts to larger pores.

Thus the present invention provides a process for controlling pore size in a catalyst composition including a heteropolyacid situated on a polyoxometallate support; wherein the heteropolyacid has the formula

$$H_{(e'-bz')}G_b(X_{k'}M_{m'-x'}M^1_{x'}M^2_{n'}O_{y'})^{-e'} \qquad (I)$$

wherein G is an element selected from Groups 1-16 or an oxy ion thereof; X is an element selected from Groups 3-16; M = molybdenum, tungsten or a combination thereof; $M^1$ = vanadium; $M^2$ is a transition metal different from M and $M^1$; z' = the charge on G; b = 0 to 12; k' = 1 to 5; m' = 5 to 20; x' = 0 to 6; n' = 0 to 3; y' = 18 to 62; and e' is the charge of the polyoxometallate anion;
wherein the polyoxometallate support has the formula

$$C_aH_{(e-az)}(X_kM_{m-x}M^1_xM^2_nO_y)^{-e} \qquad (II)$$

wherein C is a cation selected from potassium, rubidium, cesium, magnesium, calcium, strontium, barium, transition metal, actinide metal, lanthanide metal, metal oxy ion, ammonium, tetraalkylammonium, pyridinium, quinolinium, protonated aromatic amines, protonated aliphatic amines or mixtures thereof; X is an element selected from Groups 3-16 elements; M = molybdenum, tungsten or a combination thereof; $M^1$ = vanadium; $M^2$ is a transition metal different from M and $M^1$; z = the charge on C; k = 1 to 5; m = 5 to 20; n = 0 to 3; x = 0 to 6; y = 18 to 62; and e is the charge of the polyoxometallate anion;

including the step of heating the polyoxometallate support at a temperature in the range of 385° to 600° C.

It is preferred that the pore size is controlled by heating the composition at a temperature in the range of 390° to 550° C, and more preferably at a temperature in the range of 410° to 500° C.

The pore size of the polyoxometallate support may be controlled by first heating the POM followed by supporting the HPA on the POM. It is preferred that the pore size of the polyoxometallate supports are controlled by heat treating after catalyst preparation, that is, after the HPA has been supported on the POM.

When the heat treatment of the present invention is used to control the pore size of the polyoxometallate support, it is preferred that the catalyst is free of pyridine.

The following examples are presented to illustrate further various aspects of the present invention, but are not intended to limit the scope of the invention in any aspect. All reagents were of good commercial grade and were used without further purification.

<u>Example 1</u>

The heteropolyacid catalysts and polyoxometallate supports were prepared according to the following general procedures.

<u>Heteropolyacids</u>

Phosphotungstic and phosphomolybdic acids, $H_3PW_{12}O_{40}$ and $H_3PMo_{12}O_{40}$, used in the following examples were purchased commercially.

The $H_{3+x}(PM_{12-x}V_xO_{40})$-type catalysts used in the following examples were prepared according to the following general procedure illustrated for $H_5PV_2Mo_{10}O_{40}$. $H_5PV_2Mo_{10}O_{40}$ was prepared by combining 48.8 g of $NaVO_3$ in 200 g deionized water and 14.2 g $Na_2HPO_4$ in 200 g deionized water at 95° C. After cooling the mixture, 18.4 g concentrated sulfuric acid was added at which time the solution became red and an exotherm was observed. The pH decreased from 9.1 to 3.7 with the acid addition. $Na_2MoO_4$ was then added in 400 g deionized water, followed by an additional 312 g of concentrated sulfuric acid, keeping the temperature below 50° C during the exothermic acid addition. After cooling to room temperature, 1000 ml of diethyl ether was added to the mixture with vigorous stirring. The middle layer, that is the heteropolyetherate layer, was separated and the ether was evaporated. The product was dissolved in 60-80 g deionized water and crystallized. The resulting large red crystals were filtered, washed, and dried, yielding 78.53 g of desired heteropolyacid.

The heteropolyacids with extra framework promoters were generally prepared by adding aqueous solutions (e.g. 0.1 molar) of cationic promoter species, such as $VOSO_4$, $Fe(NO_3)_3$, $Cu(NO_3)_2$, to aqueous $H_3PW_{12}O_{40}$ or $H_3PMo_{12}O_{40}$ solutions (e.g. 0.1 molar) in the appropriate amounts. The samples were heated to 60° C for 30 minutes and then the water was removed by evaporation to yield the desired, solid heteropolyacids.

Typical heteropolyacids useful in the present invention are reported in Table 1.

Table 1

| Heteropolyacids |
|---|
| $H_3PMo_{12}O_{40}$ |
| $H_3PW_{12}O_{40}$ |
| $(VO)HPMo_{12}O_{40}$ |
| $(VO)_{1 \cdot 5}PMo_{12}O_{40}$ (contains $H_2SO_4$) |
| $H_5PV_2Mo_{10}O_{40}$ |
| $BiHPVMo_{11}O_{40}$ |

Polyoxometallate Supports

The polyoxometallate supports were prepared according to the following general procedure, illustrated for $Cs_3PMo_{12}O_{40}$. $Cs_3PMo_{12}O_{40}$ was prepared by adding 33.31 g of $Cs_2CO_3$ in 1225 g deionized water to 159.47 g $H_3PMo_{12}O_{40}$ in 800 g deionized water at 50° C. The addition was performed over 2 hours and the mixture was maintained at 50° C for and additional 30 minutes. After cooling to room temperature, the mixture was stirred slowly for approximately 70 hours. The water was then removed by evaporation and the resulting solid product was dried in a vacuum oven or at elevated temperature (for example 300° C), yielding approximately 150 g of the desired polyoxometallate support.

Typical polyoxometallates useful in the present invention are reported in Table 2.

Table 2

| Polyoxometallate Supports |
|---|
| $Cs_3PMo_{12}O_{40}$ |
| $Cs_3PW_{12}O_{40}$ |
| $Cs_4PVMo_{11}O_{40}$ |
| $BiPMo_{12}O_{40}$ |

Example 2

The polyoxometallate supported heteropolyacid catalysts used in the following Examples were prepared according to the following general procedure, illustrated for the preparation of $H_3PMo_{12}O_{40}/Cs_3PMo_{12}O_{40}$.

$H_3PMo_{12}O_{40}/Cs_3PMo_{12}O_{40}$ (0.02/1.0 mole ratio - approximately 10% surface coverage) was prepared by combining 0.7196 g $H_3PMo_{12}O_{40} \cdot 20\ H_2O$, 34.02 g $Cs_3PMo_{12}O_{40}$ and 315 g deionized water. After stirring at room temperature for 80 minutes, the water was removed by evaporation and the resulting solids were dried. The sample was heat treated at 150° C for one hour in air, yielding 32.69 g of the desired catalyst. The catalyst was then pelletized for packing in an oxidation reactor.

Examples of the catalysts prepared according to this procedure, as well as the approximate surface area of the support coated, are reported in Table 3.

Table 3

| Sample | Heteropolyacid | Polyoxometallate | POM Surface Area Covered (%) |
|---|---|---|---|
| 1 | $H_3PMo_{12}O_{40}$ | $Cs_3PMo_{12}O_{40}$ | 10 |
| 2 | $(VO)_{1.5}PMo_{12}O_{40}$ | $Cs_3PMo_{12}O_{40}$ | 10 |
| 3 | $H_5PV_2Mo_{10}O_{40}$ | $Cs_3PMo_{12}O_{40}$ | 10 |
| 4 | $H_3PMo_{12}O_{40}$ | $Cs_3PW_{12}O_{40}$ | 10 |
| 5 | $H_3PMo_{12}O_{40}$ | $Cs_3PMo_{12}O_{40}$ | 5 |
| 6 | $H_3PMo_{12}O_{40}$ | $Cs_3PMo_{12}O_{40}$ | 50 |
| 7 | $H_3PMo_{12}O_{40}$ | $Cs_3PMo_{12}O_{40}$ | 100 |
| 8 | $(VO)_{1.5}PMo_{12}O_{40}$ | $Cs_3PMo_{12}O_{40}$ | 100 |
| 9 | $H_5PV_2Mo_{10}O_{40}$ | $Cs_3PMo_{12}O_{40}$ | 100 |

Example 3

Four catalyst samples containing $(VO)_{1\cdot5}PMo_{12}O_{40}$ supported on $Cs_3PMo_{12}O_{40}$ were prepared. In each case, the $(VO)_{1\cdot5}PMo_{12}O_{40}$ was prepared from vanadyl sulfate without further purification. Each sample contained suf-

ficient $(VO)_{1.5}PMo_{12}O_{40}$ such that 100% of the surface area of the POM was covered. Each sample was heat treated by placing the sample in an oven at room temperature and heating the sample to the final heat treatment temperature at a rate of about 2° C per minute. Each sample was held at the final heat treatment temperature for 2 hours. One sample served as a control and was heat treated at 300° C, which is the typical drying or calcining temperature of a HPA/POM catalyst. The other samples were heat treated at 420°, 450° or 500° C. In each case, either the POM was heat treated before being coated by the HPA ("pre-coat") or the catalyst including the HPA supported on the POM was heat treated ("post-coat").

After heat treatment, each catalyst was placed in a catalytic reactor at room temperature under air, propane was fed into the reactor and the reactor was heated to 380° C. The yield of acrylic acid ("AA") was measured for each catalyst at approximately 30% conversion of the propane. These results are reported in Table 4.

Table 4

| Temperature (° C) | Coating Conditions | AA Yield at 30% Conversion |
|---|---|---|
| 300 * | post-coat | 2.5 |
| 420 | post-coat | 4.9 |
| 450 | post-coat | 6.4 |
| 500 | pre-coat | 3.2 |

* Control

The above data clearly show that the yields of acrylic acid greatly increased when the catalysts were heat treated at high temperatures. The data also show that heat treating the combined HPA/POM catalysts further increases the yield of acrylic acid.

Example 4

Two samples of catalysts containing $(VO)_{1.5}PMo_{12}O_{40}$ supported on $Cs_3PMo_{12}O_{40}$ were prepared. Each sample contained sufficient $(VO)_{1.5}PMo_{12}O_{40}$ such that 100% of the surface area of the POM was covered. One sample served as a control and was heat treated at 300° C as described in Example 3. The other sample was heat treated at 420° C in situ in a catalytic reactor. The heat treated sample was placed in a catalytic reactor at room temperature under air, propane was fed into the reactor and the temperature was increased to 420° C. The reactor was held at this temperature for less than 10 minutes, and then cooled to 380° C and the reaction continued. The yield of acrylic acid was measured at 30% conversion of the propane. The control sample was also used to catalyze the conversion of propane to acrylic acid, as described in Example 3. The data for the control sample and the in situ heat treated sample are reported in Table 5.

Table 5

| Temperature (° C) | Coating Conditions | AA Yield at 30% Conversion |
|---|---|---|
| 300 * | post-coat | 2.5 |
| 420 | post-coat | 6.4 |

* Control

The above data clearly show that in situ heat treatment of a HPA/POM catalyst is very effective at increasing the yield of acrylic acid from propane.

Example 5

HPA/POM catalysts were prepared by combining a heteropolyacid (30 wt%) on $Cs_3PMo_{12}O_{40}$ support. The specific heteropolyacids used are reported in the following table. One sample which was not pretreated with an amine was heat treated at 275° C and served as a comparative. The remaining samples were heat treated at either 420° or

440° C. Some of the samples were pretreated with an amine in an amount from 0.25 to 1.0 equivalents, based on the amount of the heteropolyacid. The amines used were pyridine ("PYR"), quinoline ("QUIN") and bipyridine ("BIPYR"). After heat treating, the catalysts were then used to catalyze the oxidation of propane to acrylic acid. The oxidations were carried out by passing propane, air and nitrogen mixture (1.76/15.8/9.6ml/min) over 4 ml of the catalyst at 375° to 380° C. The yield of acrylic acid, propane conversion and acrylic acid selectivity are reported in Table 6.

Table 6

| Heteropolyacid Catalyst | Heat Treatment Temp.(°C) | AA Yield (%) | $C_3$ Conversion (%) | AA Selectivity (%) | Amine (equivalents) |
|---|---|---|---|---|---|
| $H_2(VO)_{0\cdot5}PMo_{12}O_{40}$ * | 275 | 2.1 | 9 | 22.7 | none |
| $H_2(VO)_{0\cdot5}PMo_{12}O_{40}$ | 420 | 7.8 | 34 | 22.9 | PYR (0.5) |
| $H_2(VO)_{0\cdot5}PMo_{12}O_{40}$ | 420 | 8.2 | 46 | 17.9 | QUIN (0.5) |
| $H_2(VO)_{0.5}PMo_{12}O_{40}$ | 420 | 7.6 | 42 | 18.1 | BIPYR (0.25) |
| $H_2(VO)_{0.5}PMo_{12}O_{40}$ | 420 | 10.8 | 50 | 21.5 | BIPYR (1.0) |
| $H_2(VO)_{0\cdot5}PMo_{12}O_{40}$ | 440 | 10.2 | 45 | 22.7 | none |
| $H(VO)PMo_{12}O_{40}$ | 440 | v. low | -- | -- | BIPYR (1.0) |

* Comparative

The above data clearly show that heat treating the HPA/POM catalysts provides improved yield, conversion and selectivity of acrylic acid as compared to non-heat treated catalysts. The above data further show that heat treated catalysts that were also pretreated with amines do not improve the yields and selectivity of acrylic acid over those without such amine pretreatment.

**Claims**

**1.** A catalyst composition comprising a heteropolyacid situated on a polyoxometallate support; wherein the heteropolyacid has the formula

$$H_{(e'-bz')}G_b(X_{k'}M_{m'-x'}M^1_{x'}M^2_{n'}O_{y'})^{-e'} \qquad (I)$$

wherein G is an element selected from Groups 1-16 or an oxy ion thereof; X is an element selected from Groups 3-16; M = molybdenum, tungsten or a combination thereof; $M^1$ = vanadium; $M^2$ is a transition metal different from M and $M^1$; z' = the charge on G; b = 0 to 12; k' = 1 to 5; m' = 5 to 20; x' = 0 to 6; n' = 0 to 3; y' = 18 to 62; and e' is the charge of the polyoxometallate anion;
wherein the polyoxometallate support has the formula

$$C_aH_{(e-az)}(X_kM_{m-x}M^1_xM^2_nO_y)^{-e} \qquad (II)$$

wherein C is a cation selected from potassium, rubidium, cesium, magnesium, calcium, strontium, barium, transition metal, actinide metal, lanthanide metal, metal oxy ion, ammonium, tetraalkylammonium, pyridinium, quinolinium, protonated aromatic amines, protonated aliphatic amines or mixtures thereof; X is an element selected from Groups 3-16 elements; M = molybdenum, tungsten or a combination thereof; $M^1$ = vanadium; $M^2$ is a transition metal different from M and $M^1$; z = the charge on C; k = 1 to 5; m = 5 to 20; n = 0 to 3; x = 0 to 6; y = 18 to 62; and e is the charge of the polyoxometallate anion; and
wherein the polyoxometallate support is heat treated at a temperature in the range of 385° to 600° C.

**2.** The composition of claim 1 wherein the polyoxometallate support comprises cesium salt of heteropolyacid having the formula $Cs_{3+x}(PM_{12-x}V_xO_{40})$, where M is molybdenum or tungsten, and x is 0 to 3.

**3.** The composition of claim 2 wherein the polyoxometallate support comprises $Cs_3(PMo_{12}O_{40})$, $Cs_4(PMo_{11}VO_{40})$, $Cs_5(PMo_{10}V_2O_{40})$, $Cs_6(PMo_9V_3O_{40})$, $Cs_3(PW_{12}O_{40})$, $Cs_4(PW_{11}VO_{40})$, $Cs_5(PW_{10}V_2O_{40})$, $Cs_6(PW_9V_3O_{40})$ or

combinations thereof.

**4.** The composition of claim 3 wherein the polyoxometallate support comprises $Cs_3(PMo_{12}O_{40})$ or $Cs_3(PW_{12}O_{40})$.

**5.** The composition of claim 1 wherein the heteropolyacid comprises $H_3PMo_{12}O_{40}$, $H_3PW_{12}O_{40}$, $(VO)_{1.5}PMo_{12}O_{40}$, $(VO)_{1.5}PW_{12}O_{40}$, $(TiO)_{1.5}PMo_{12}O_{40}$, $H(VO)PMo_{12}O_{40}$, $H(VO)PW_{12}O_{40}$, $H_6PV_3Mo_9O_{40}$, $H_5PV_2Mo_{10}O_{40}$, $H_5PV_2W_{10}O_{40}$, $H_4PVMo_{11}O_{40}$, $H_4PVW_{11}O_{40}$, $RhPMo_{12}O_{40}$, $BiPMo_{12}O_{40}$, $HCrPVMo_{11}O_{40}$, $HBiPVMo_{11}O_{40}$ or combinations thereof.

**6.** The composition of claim 5 wherein the heteropolyacid comprises $H_3PMo_{12}O_{40}$, $H_3PW_{12}O_{40}$, $(VO)_{1.5}PMo_{12}O_{40}$, or $H(VO)PMo_{12}O_{40}$.

**7.** The composition of claim 1 wherein the heteropolyacid comprises $H_3PMo_{12}O_{40}$, $H_3PW_{12}O_{40}$, $(VO)_{1.5}PMo_{12}O_{40}$, $(VO)_{1.5}PW_{12}O_{40}$, $(TiO)_{1.5}PMo_{12}O_{40}$, $H(VO)PMo_{12}O_{40}$, $H(VO)PW_{12}O_{40}$, $H_6PV_3Mo_9O_{40}$, $H_5PV_2Mo_{10}O_{40}$, $H_5PV_2W_{10}O_{40}$, $H_4PVMo_{11}O_{40}$, $H_4PVW_{11}O_{40}$, $RhPMo_{12}O_{40}$, $BiPMo_{12}O_{40}$, $HCrPVMo_{11}O_{40}$, $HBiPVMo_{11}O_{40}$ or combinations thereof; and wherein the polyoxometallate support comprises cesium salt of heteropolyacid having the formula $Cs_{3+x}(PM_{12-x}V_xO_{40})$, where M is molybdenum or tungsten, and x is 0 to 3.

**8.** The composition of claim 1 wherein both the heteropolyacid and polyoxometallate support are free of pyridine.

**9.** The composition of claim 1 wherein the composition is heat treated at a temperature in the range of 390° to 470° C.

**10.** The composition of claim 9 wherein the composition is heat treated at a temperature in the range of 410° to 450° C.

**11.** The composition of claim 1 wherein the polyoxometallate support is heat treated before the heteropolyacid is situated on the support.

**12.** The composition of claim 1 wherein the polyoxometallate support is heat treated after the heteropolyacid is situated on the support.

**13.** A process for preparing a catalyst composition comprising a heteropolyacid on a polyoxometallate support; wherein the heteropolyacid has the formula

$$H_{(e'-bz')}G_b(X_{k'}M_{m'-x'}M^1_{x'}M^2_{n'}O_{y'})^{-e'} \qquad (I)$$

wherein G is an element selected from Groups 1-16 or an oxy ion thereof; X is an element selected from Groups 3-16; M = molybdenum, tungsten or a combination thereof; $M^1$ = vanadium; $M^2$ is a transition metal different from M and $M^1$; z' = the charge on G; b = 0 to 12; k' = 1 to 5; m' = 5 to 20; x' = 0 to 6; n' = 0 to 3; y' = 18 to 62; and e' is the charge of the polyoxometallate anion;
wherein the polyoxometallate support has the formula

$$C_aH_{(e-az)}(X_kM_{m-x}M^1_xM^2_nO_y)^{-e} \qquad (II)$$

wherein C is a cation selected from potassium, rubidium, cesium, magnesium, calcium, strontium, barium, transition metal, actinide metal, lanthanide metal, metal oxy ion, ammonium, tetraalkylammonium, pyridinium, quinolinium, protonated aromatic amines, protonated aliphatic amines or mixtures thereof; X is an element selected from Groups 3-16; M is as defined above; $M^1$ = vanadium; $M^2$ is a transition metal different from M and $M^1$; z = the charge on C; k = 1 to 5; m = 5 to 20; n = 0 to 3; x = 0 to 6; y = 18 to 62; and e is the charge of the polyoxometallate anion;

comprising the step of heating the polyoxometallate support at a temperature in the range of 385° to 600° C.

**14.** The process of claim 13 wherein the polyoxometallate support comprises cesium salt of heteropolyacid having the formula $Cs_{3+x}(PM_{12-x}V_xO_{40})$, where M is molybdenum or tungsten, and x is 0 to 3.

**15.** The process of claim 14 wherein the polyoxometallate support comprises $Cs_3(PMo_{12}O_{40})$, $Cs_4(PMo_{11}VO_{40})$,

$Cs_5(PMo_{10}V_2O_{40})$, $Cs_6(PMo_9V_3O_{40})$, $Cs_3(PW_{12}O_{40})$, $Cs_4(PW_{11}VO_{40})$, $Cs_5(PW_{10}V_2O_{40})$, $Cs_6(PW_9V_3O_{40})$ or combinations thereof.

**16.** The process of claim 13 wherein the heteropolyacid comprises $H_3PMo_{12}O_{40}$, $H_3PW_{12}O_{40}$, $(VO)_{1.5}PMo_{12}O_{40}$, $(VO)_{1.5}PW_{12}O_{40}$, $(TiO)_{1.5}PMo_{12}O_{40}$, $H(VO)PMo_{12}O_{40}$, $H(VO)PW_{12}O_{40}$, $H_6PV_3Mo_9O_{40}$, $H_5PV_2Mo_{10}O_{40}$, $H_5PV_2W_{10}O_{40}$, $H_4PVMo_{11}O_{40}$, $H_4PVW_{11}O_{40}$, $RhPMo_{12}O_{40}$, $BiPMo_{12}O_{40}$, $HCrPVMo_{11}O_{40}$, $HBiPVMo_{11}O_{40}$ or combinations thereof.

**17.** The process of claim 13 wherein both the heteropolyacid and polyoxometallate support are free of pyridine.

**18.** A process for preparing unsaturated carboxylic acids comprising the steps of contacting an alkane with an oxidizing agent and a catalyst composition comprising a heteropoly acid on a polyoxometallate support; wherein the heteropoly acid has the formula

$$H_{(e'-bz')}G_b(X_{k'}M_{m'-x'}M^1_{x'}M^2_{n'}O_{y'})^{-e'} \qquad (I)$$

wherein G is an element selected from Groups 1-16 or an oxy ion thereof; X is an element selected from Groups 3-16; M = molybdenum, tungsten or a combination thereof; $M^1$ = vanadium; $M^2$ is a transition metal different from M and $M^1$; z' = the charge on G; b = 0 to 12; k' = 1 to 5; m' = 5 to 20; x' = 0 to 6; n' = 0 to 3; y' = 18 to 62; and e' is the charge of the polyoxometallate anion;
wherein the polyoxometallate support has the formula

$$C_aH_{(e-az)}(X_kM_{m-x}M^1_xM^2_nO_y)^{-e} \qquad (II)$$

wherein C is a cation selected from potassium, rubidium, cesium, magnesium, calcium, strontium, barium, transition metal, actinide metal, lanthanide metal, metal oxy ion, ammonium, tetraalkylammonium,pyridinium, quinolinium, protonated aromatic amines, protonated aliphatic amines or mixtures thereof; X is an element selected from Groups 3-16 elements; M = molybdenum, tungsten or a combination thereof; $M^1$ = vanadium; $M^2$ is a transition metal different from M and $M^1$; z = the charge on C; k = 1 to 5; m = 5 to 20; n = 0 to 3; x = 0 to 6; y = 18 to 62; and e is the charge of the polyoxometallate anion;
wherein the heteropolyacid is present in an amount such that 100% of the surface area of the polyoxometallate is covered; and
wherein the polyoxometallate support is heat treated at a temperature in the range of 385° to 600° C prior to contacting the alkane.

**19.** The process of claim 18 wherein the polyoxometallate support comprises cesium salt of heteropolyacid having the formula $Cs_{3+x}(PM_{12-x}V_xO_{40})$, where M is molybdenum or tungsten, and x is 0 to 3.

**20.** The process of claim 19 wherein the polyoxometallate support comprises $Cs_3(PMo_{12}O_{40})$, $Cs_4(PMo_{11}VO_{40})$, $Cs_5(PMo_{10}V_2O_{40})$, $Cs_6(PMo_9V_3O_{40})$, $Cs_3(PW_{12}O_{40})$, $Cs_4(PW_{11}VO_{40})$, $Cs_5(PW_{10}V_2O_{40})$, $Cs_6(PW_9V_3O_{40})$ or combinations thereof.

**21.** The process of claim 18 wherein the heteropolyacid comprises $H_3PMo_{12}O_{40}$, $H_3PW_{12}O_{40}$, $(VO)_{1.5}PMo_{12}O_{40}$, $(VO)_{1.5}PW_{12}O_{40}$, $(TiO)_{1.5}PMo_{12}O_{40}$, $H(VO)PMo_{12}O_{40}$, $H(VO)PW_{12}O_{40}$, $H_6PV_3Mo_9O_{40}$, $H_5PV_2Mo_{10}O_{40}$, $H_5PV_2W_{10}O_{40}$, $H_4PVMo_{11}O_{40}$, $H_4PVW_{11}O_{40}$, $RhPMo_{12}O_{40}$, $BiPMo_{12}O_{40}$, $HCrPVMo_{11}O_{40}$, $HBiPVMo_{11}O_{40}$ or combinations thereof.

**22.** The process of claim 18 wherein both the heteropolyacid and polyoxometallate support are free of pyridine.

**23.** A process for preparing alkenes comprising the steps of contacting an alkane with an oxidizing agent with a catalyst composition comprising a heteropolyacid on a polyoxometallate support; wherein the heteropolyacid has the formula

$$H_{(e'-bz')}G_b(X_{k'}M_{m'-x'}M^1_{x'}M^2_{n'}O_{y'})^{-e'} \quad (I)$$

wherein G is an element selected from Groups 1-16 or an oxy ion thereof; X is an element selected from Groups 3-16; M = molybdenum, tungsten or a combination thereof; $M^1$ = vanadium; $M^2$ is a transition metal different from M and $M^1$; z' = the charge on G; b = 0 to 12; k' = 1 to 5; m' = 5 to 20; x' = 0 to 6; n' = 0 to 3; y'= 18 to 62; and e' is the charge of the polyoxometallate anion;
wherein the polyoxometallate support has the formula

$$C_aH_{(e-az)}(X_kM_{m-x}M^1_xM^2_nO_y)^{-e} \quad (II)$$

wherein C is a cation selected from potassium, rubidium, cesium, magnesium, calcium, strontium, barium, transition metal, actinide metal, lanthanide metal, metal oxy ion, ammonium, tetraalkylammonium,pyridinium, quinolinium, protonated aromatic amines, protonated aliphatic amines or mixtures thereof; X is an element selected from Groups 3-16 elements; M = molybdenum, tungsten or a combination thereof $M^1$ = vanadium; $M^2$ is a transition metal different from M and $M^1$; z = the charge on C; k = 1 to 5; m = 5 to 20; n = 0 to 3; x = 0 to 6; y = 18 to 62; and e is the charge of the polyoxometallate anion;
wherein the heteropolyacid is present in an amount such that less than 100% of the surface area of the polyoxometallate is covered; and
wherein the polyoxometallate support is heat treated at a temperature in the range of 385° to 600° C prior to contacting the alkane.

**24.** The process of claim 23 wherein the polyoxometallate support comprises cesium salt of heteropolyacid having the formula $Cs_{3+x}(PM_{12-x}V_xO_{40})$, where M is molybdenum or tungsten, and x is 0 to 3.

**25.** The process of claim 24 wherein the polyoxometallate support comprises $Cs_3(PMo_{12}O_{40})$, $Cs_4(PMo_{11}VO_{40})$, $Cs_5(PMo_{10}V_2O_{40})$, $Cs_6(PMo_9V_3O_{40})$, $Cs_3(PW_{12}O_{40})$, $Cs_4(PW_{11}VO_{40})$, $Cs_5(PW_{10}V_2O_{40})$, $Cs_6(PW_9V_3O_{40})$ or combinations thereof.

**26.** The process of claim 23 wherein the heteropolyacid comprises $H_3PMo_{12}O_{40}$, $H_3PW_{12}O_{40}$, $(VO)_{1.5}PMo_{12}O_{40}$, $(VO)_{1.5}PW_{12}O_{40}$, $(TiO)_{1.5}PMo_{12}O_{40}$, $H(VO)PMo_{12}O_{40}$, $H(VO)PW_{12}O_{40}$, $H_6PV_3Mo_9O_{40}$, $H_5PV_2Mo_{10}O_{40}$, $H_5PV_2W_{10}O_{40}$, $H_4PVMo_{11}O_{40}$, $H_4PVW_{11}O_{40}$, $RhPMo_{12}O_{40}$, $BiPMo_{12}O_{40}$, $HCrPVMo_{11}O_{40}$, $HBiPVMo_{11}O_{40}$ or combinations thereof.

**27.** The process of claim 23 wherein both the heteropolyacid and polyoxometallate support are free of pyridine.

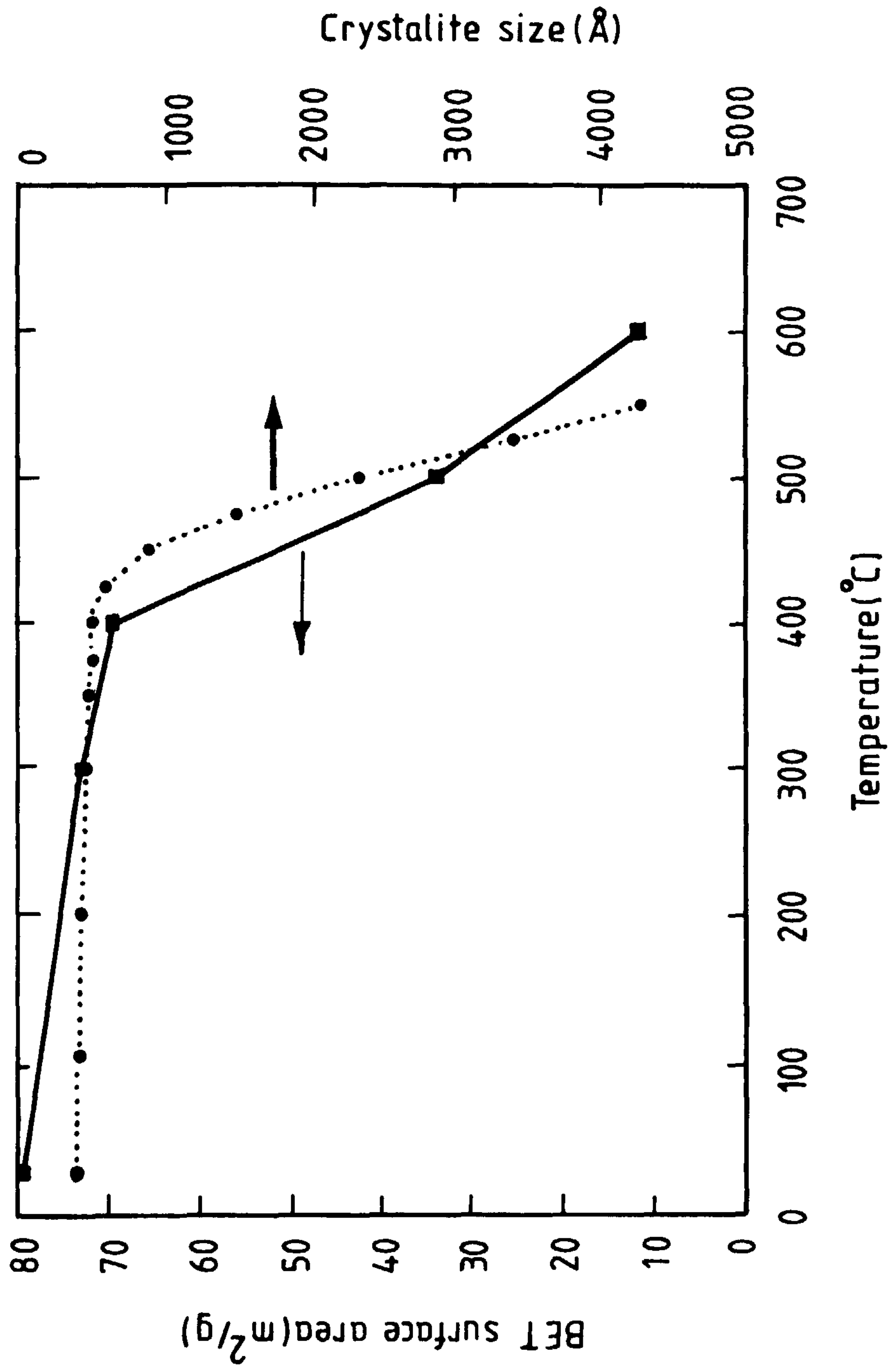

Crystalite size (Å)
0
1000
2000
3000
4000
5000
0
100
200
300
400
500
600
700
Temperature (°C)
80
70
60
50
40
30
20
10
0
BET surface area (m2/g)


Fig. 1.

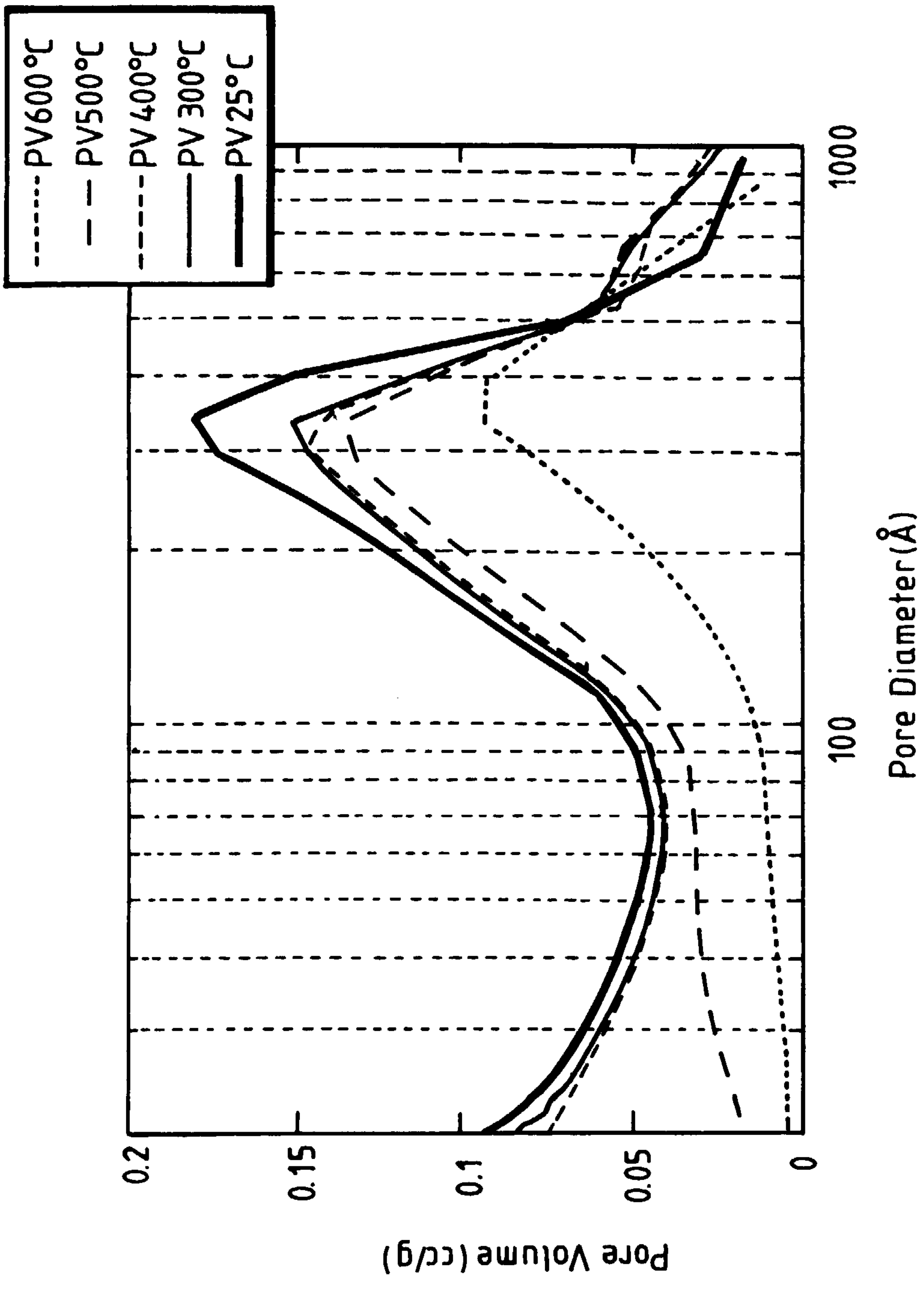
PV600°C
PV500°C
PV400°C
PV 300°C
PV25°C
0.2
0.15
0.1
0.05
0
Pore Volume (cc/g)
100
1000
Pore Diameter (Å)

Fig. 2.

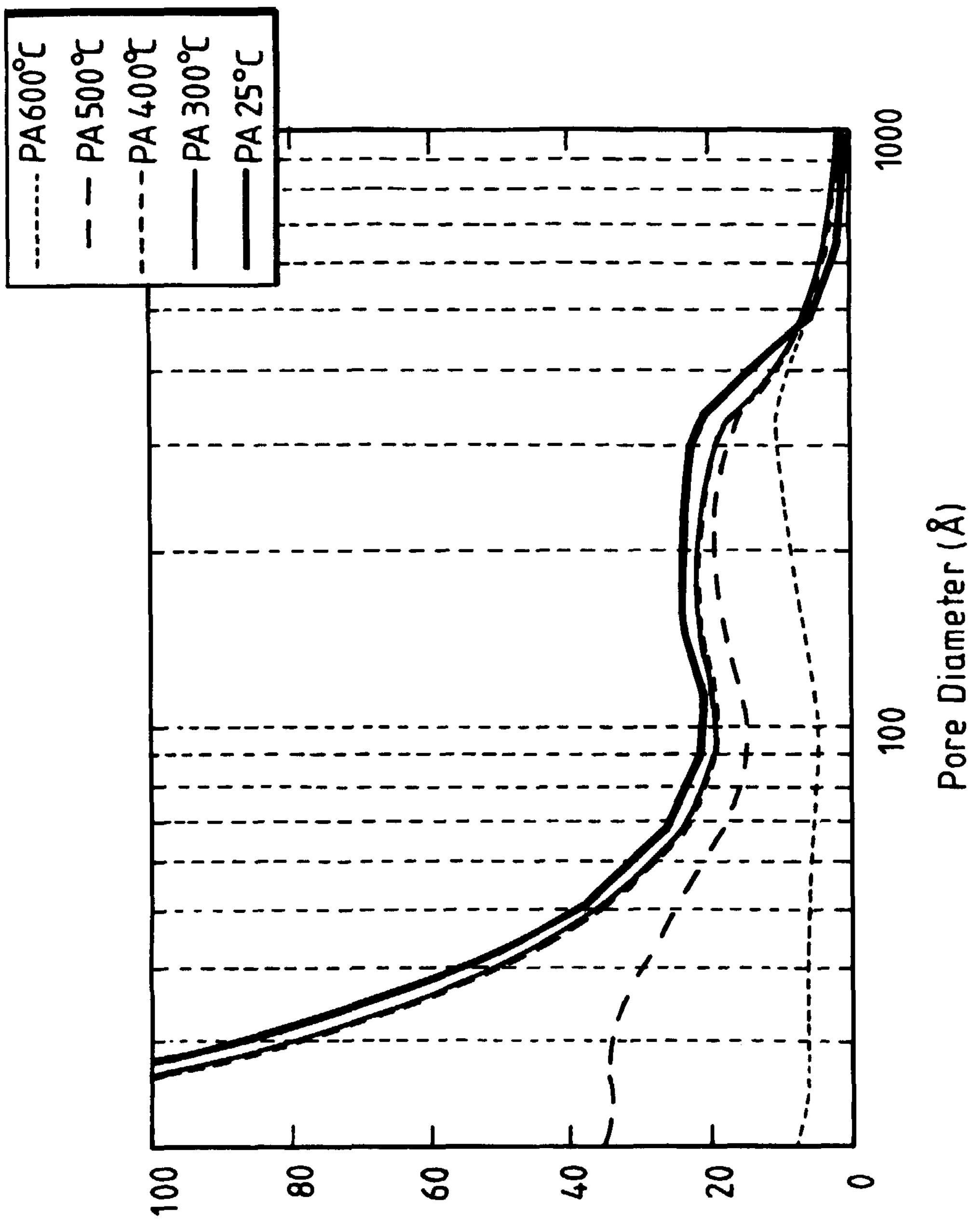
PA600°C
PA500°C
PA400°C
PA300°C
PA25°C
Pore Area (sq.m/g)
100
80
60
40
20
0
Pore Diameter (Å)
100
1000

Fig. 3.

European Patent Office

# EUROPEAN SEARCH REPORT

Application Number
EP 00 30 6729

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| P,X | WO 00 09262 A (ROHM & HAAS ;SUNOCO INC R & M (US)) 24 February 2000 (2000-02-24) * claims 1-29 * | 1-7, 13-21 | B01J27/188 C07C51/215 C07C5/42 |
| P,X | US 5 990 348 A (LYONS JAMES E ET AL) 23 November 1999 (1999-11-23) * claims 1,21; tables 1-4 * | 1-7, 13-21 | |
| A | EP 0 771 781 A (SUN CO INC R & M) 7 May 1997 (1997-05-07) | | |
| A | EP 0 713 723 A (BP CHEM INT LTD) 29 May 1996 (1996-05-29) | | |
| A | US 5 919 725 A (PAES JOSE AGUSTO DA COSTA ET AL) 6 July 1999 (1999-07-06) | | |
| A | EP 0 704 240 A (BP CHEM INT LTD) 3 April 1996 (1996-04-03) | | |

TECHNICAL FIELDS SEARCHED (Int.Cl.7)

B01J
C07C

The present search report has been drawn up for all claims

| Place of search | Date of completion of the search | Examiner |
|---|---|---|
| THE HAGUE | 14 November 2000 | Thion, M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 00 30 6729

This annex lists the patent family members relating to the patent documents cited in the above-mentioned European search report.
The members are as contained in the European Patent Office EDP file on
The European Patent Office is in no way liable for these particulars which are merely given for the purpose of information.

14-11-2000

| Patent document cited in search report | Publication date | Patent family member(s) | Publication date |
|---|---|---|---|
| WO 0009262 A | 24-02-2000 | US 6060419 A | 09-05-2000 |
| US 5990348 A | 23-11-1999 | US 6060419 A | 09-05-2000 |
| EP 0771781 A | 07-05-1997 | US 5705685 A | 06-01-1998 |
| | | CA 2187413 A | 01-05-1997 |
| | | DE 69607562 D | 11-05-2000 |
| | | JP 9183748 A | 15-07-1997 |
| EP 0713723 A | 29-05-1996 | CA 2163324 A | 24-05-1996 |
| | | CN 1128178 A | 07-08-1996 |
| | | JP 8224480 A | 03-09-1996 |
| | | SG 46963 A | 20-03-1998 |
| | | US 5616815 A | 01-04-1997 |
| US 5919725 A | 06-07-1999 | WO 9513869 A | 26-05-1995 |
| | | US 5866739 A | 02-02-1999 |
| EP 0704240 A | 03-04-1996 | CA 2158527 A | 27-03-1996 |
| | | CN 1130103 A | 04-09-1996 |
| | | DE 69513608 D | 05-01-2000 |
| | | DE 69513608 T | 27-04-2000 |
| | | JP 8192047 A | 30-07-1996 |
| | | SG 32502 A | 13-08-1996 |
| | | US 5714429 A | 03-02-1998 |
| | | US 5684216 A | 04-11-1997 |

EPO FORM P0459

For more details about this annex : see Official Journal of the European Patent Office, No. 12/82